(19) Europäisches Patentamt
European Patent Office
Office européen des brevets

(11) EP 1 197 216 B1

(12) EUROPÄISCHE PATENTSCHRIFT

(45) Veröffentlichungstag und Bekanntmachung des Hinweises auf die Patenterteilung:
**23.06.2004 Patentblatt 2004/26**

(51) Int Cl.[7]: **A61K 31/716**, C08B 37/00, A61K 7/48

(21) Anmeldenummer: **01128699.4**

(22) Anmeldetag: **17.07.1997**

# (54) Pharmazeutische oder kosmetische Zusammensetzung enthaltend polymere Glucanäther-Derivate

Pharmaceutical or cosmetic composition containing polyglucan ether derivatives

Composition pharmaceutique ou cosmétique contenant des dérivés d'éthers de polyglucane

(84) Benannte Vertragsstaaten:
**CH DE DK FI FR GB LI SE**

(30) Priorität: **19.07.1996 DE 19629117**

(43) Veröffentlichungstag der Anmeldung:
**17.04.2002 Patentblatt 2002/16**

(62) Dokumentnummer(n) der früheren Anmeldung(en) nach Art. 76 EPÜ:
**97112307.0 / 0 819 703**

(73) Patentinhaber: **Mibelle AG Cosmetics**
**5033 Buchs (CH)**

(72) Erfinder:
- **Zülli, Fred, Dr.sc.nat.**
  **5024 Küttingen (CH)**
- **Suter, Franz**
  **5312 Böttingen (CH)**

(74) Vertreter: **Lau-Loskill, Philipp, Dipl.-Phys.**
**Patentanwalt**
**Berger Dorfstrasse 35**
**41189 Mönchengladbach (DE)**

(56) Entgegenhaltungen:
**EP-A- 0 397 880**
**DE-A- 2 152 059**
**DE-A- 4 431 251**
**US-A- 4 454 315**

- **F. ZÜLLI ET AL.: "CM-Glucan a new yeast polysaccharide for cosmetic use" COSMETICS AND TOILETRIES MANUFACTURE WORLDWIDE, 1994, Seite 131,133,134,136 XP002090001**
- **F. ZÜLLI ET AL.: "Photoprotective effects of CM-glucan on cultured human skin cells" EURO COSMETICS, Bd. 1995, Nr. 11, 1995, Seite 46-50 XP002090000**
- **PATENT ABSTRACTS OF JAPAN vol. 015, no. 474 (C-0890), 3. Dezember 1991 (1991-12-03) & JP 03 204804 A (MITSUI TOATSU CHEM. INC.)**
- **S. DEMLEITNER ET AL.: "Synthesis and antitumour activity of sulfoalkyl derivatives of curdlan and lichenan" CARBOHYDRATE RESEARCH, Bd. 226, 30. März 1992 (1992-03-30), Seite 247-252 XP000258120**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine polymere Glucanäther-Derivate enthaltende Zusammensetzung mit den Merkmalen des Oberbegriffs des Patentanspruchs 1.

Glucan ist eine 1,3-beta-Polyglukose und wird durch einen alkalischen sowie sauren Aufschluß aus Hefezellen gewonnen, wie dieser beispielsweise in den US 5,397,773 A, der US-5,250,436 A oder der US-5,223,491 A beschrieben ist.

Als erfindungsgemäße Zusammensetzungen gelten solche, die zwischen 0,002 Ges.% und 8 Gew.% des erfindungsgemäßen Glucanäther-Derivates, zwischen 0 Gew.% und 3 Gew.% eines Alkohols (ausgenommen Alkylenglykol), insbesondere eines $C_1$ -$C_4$ -Alkohols und vorzugsweise Ethanol, zwischen 0,5 Gew.% und 15 Gew.% eines Alkylenglykols, vorzugsweise eines mittelviskosen Propylenglykols, zwischen 1 Gew.% und 3 Gew.% eines Paraffins, vorzugsweise eines flüssigen Paraffins, zwischen 5,5 Gew.% und 9,5 Gew.% sonstige Zusätze sowie zwischen 92,988 Gew.% und 75 Gew.% Wasser aufweist. Hierbei besitzt eine derartige Zusammensetzung dann eine gel- bis cremeartige Konsistenz auf, so daß sie dementsprechend einfach und unproblematisch topisch zu applizieren ist. Auch konnte festgestellt werden, daß die zuvor angesprochene Zusammensetzung sehr schnell nach dem Auftragen in die Haut einzieht, so daß ein unerwünschtes Nachfetten der Zusammensetzung bereits nach wenigen Sekunden bis einigen Minuten nach Applikation der Zusammensetzung verschwunden ist. Als sonstige Zusätze weist die zuvor angesprochene Zusammensetzung beispielsweise Verfestigungsmittel, so insbesondere hydrierte Polyisobutene, Konservierungsmittel, so beispielsweise Phenoxyethanol und/oder Imidazolidinyl-Harnstoff, Verdickungsmittel, so beispielsweise Stearinsäure und/oder Palmitinsäure, und Duftstoffe auf.

Die erfindungsgemäßen Zusammensetzungen enthalten neben den üblichen Inhaltsstoffen als Wirkstoff mindestens ein polymeres Glucanäther-Derivat, das neben Monomereneinheiten der Formel I auch solche Monomereneinheiten der allgemeinen Formel II aufweist,

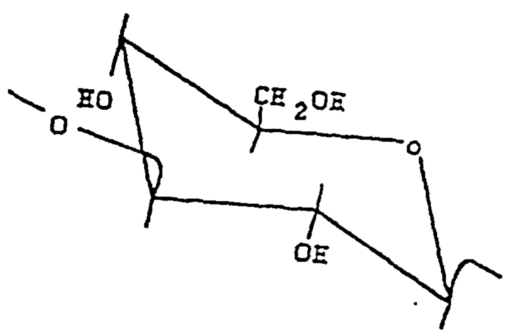

(Formel I)

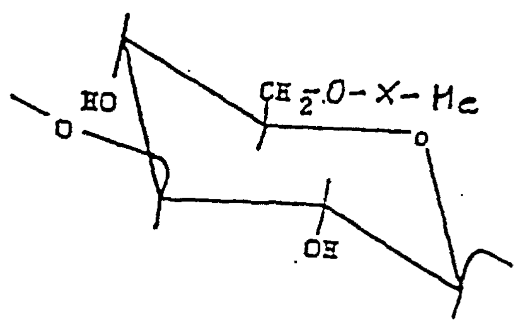

(Formel II)

wobei die Monomereneinheiten der Formel I mit den Monomereneinheiten der Formel II über eine 1,3-beta-glykosidische Bindung verknüpft sind und wobei in Formel II

X eine $-CH_2-COO$-Gruppe, eine $-CH_2-CH_2-COO$-Gruppe, eine

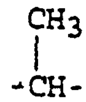

COO-Gruppe und/oder eine $-CH_2-CH_2-SO_3$-Gruppe und Me Wasserstoff, ein Alkali- und/oder ein Erdalkalimetall bedeuten. Dieses Carbox methyl-glucan wird vorzugsweise so hergestellt, wie dies in der EP 0 819 703 A2 bansprücht ist.

Überraschend konnte festgestellt werden, daß die erfindungsgemäße Zusammensetzung, die eine zur topischen Anwendung geeignete Darreichungsform besitzt, hervorragend zur Prophylaxe und/oder Therapie von Hautreizungen bzw. Hauterkrankungen, die ihre Ursache in einem oxidativen Streß in der Haut haben, geeignet ist. Derartige Hauterkrankungen bzw. Hautreizungen werden durch UVA-Strahlen, oxidierenden Chemikalien, durch Sonneneinstrahlungen oder sonstigen umweltbedingten Beeinträchtigungen der Haut hervorgerufen. Auch treten derartige Hautveränderungen im Laufe der Hautalterung auf, so daß die erfindungsgemäße pharmazeutische und/oder kosmetische Zubereitung der Hautalterung entgegenwirkt.

Die polymeren Glucanäther-Derivate besitzen eine hervorragende Wasserlöslichkeit, wobei abhängig von der Gruppe X und der Gruppe Me in Formel II diese Wasserlöslichkeit um den Faktor 100 bis etwa 500 größer ist als die Wasserlöslichkeit von Glucan. Diese extrem große Wasserlöslichkeit der erfindungsgemäßen Glucanäther-Derivate, die absolut gesehen bis zu etwa 80 g pro Liter Wasser beträgt, führt dazu, daß die erfindungsgemäßen Glucanäther-Derivate ausgezeichnet als Wirkstoffe in pharmazeutischen und/oder kosmetischen Zubereitungen eingesetzt werden können, wobei derartige pharmazeutische bzw. kosmetische Zubereitungen vorzugsweise topisch appliziert werden und z.B. zur Prophylaxe und/oder Therapie von Hauterkrankungen, Hautverletzungen, Hautreizungen sowie Hautalterung, verwendet werden, wie dies nachfolgend noch detailliert erklärt ist.

Vorzugsweise besitzen die Glucanäther-Derivate ein Molekulargewicht zwischen 20.000 und 2.000.000, insbesondere zwischen 50.000 und 500.000.

Die gute Wasserlöslichkeit der Glucanäther-Derivate hängt nicht nur von der vorstehend in der Formel II mit X und Me bezeichneten Gruppen sondern auch von dem Durchschnittssubstitutionsgrad ab, wobei bei den Glucanäther-Derivate der mittlere Durchschnittssubstitutionsgrad zwischen 0,4 und 0,9, insbesondere zwischen 0,6 und 0,8, variiert. Insbesondere dann, wenn der mittlere Durchschnittssubstitutionsgrad 0,75 beträgt und wenn in der Formel II X eine $-CH_2-COO$-Gruppe und Me Natrium darstellt, besitzt ein derartiges Carboxymethylglucan-Natrium eine ausgezeichnete Wasserlöslichkeit, die im Bereich von etwa 40 g/l Wasser liegt. Von daher wird dieses spezielle Glucanäther-Derivat bevorzugt zur Herstellung von topisch applizierbaren pharmazeutischen und/oder kosmetischen Zusammensetzungen, wie diese nachfolgend noch im Detail beschrieben sind, eingesetzt.

Grundsätzlich können bei den Glucanäther-Derivaten der in der Formel II mit Me gekennzeichnete Rest Wasserstoff, ein Alkalimetall und/oder ein Erdalkalimetall bedeuten. Besonders dann, wenn in der Formel II Me für ein Alkalimetall, vorzugsweise für Natrium, steht, weist ein derartiges konkretes Glucanäther-Derivat eine hohe Hautverträglichkeit auf, so daß dieses Glucanäther-Derivat selbst bei empfindlichen Anwendern keine Hautreizungen verursacht.

Dies trifft auch auf solche Glucanäther-Derivate zu, bei denen in der vorstehend wiedergegebenen Formel II X für eine $-CH_2-COO$-Gruppe und/oder für eine $-CH_2-CH_2-SO_3$-Gruppe steht, wobei festgestellt werden konnte, daß diese konkreten Glucanäther-Derivate neben einer guten Wasserlöslichkeit und einer hohen Hautverträglichkeit desweiteren ausgezeichnete Wirkstoffeigenschaften besitzen, so daß derartige Glucanäther-Derivate bevorzugt in topisch applizierbaren pharmazeutischen und/oder kosmetischen Zubereitungen eingesetzt werden, die zur Prophylaxe und/oder Therapie von Hauterkrankungen, Hautverletzungen, Hautreizungen, sowie Hautalterung verwendet werden.

Vorstehend ist im Zusammenhang mit der erfindungsgemäßen Zusammensetzung beschrieben, daß diese hervorragend zur Prophylaxe und/oder Therapie von Hautreizungen bzw. Hauterkrankungen, die ihre Ursache in einem oxidativen Streß in der Haut haben, geeignet ist.

Überraschend konnte desweiteren festgestellt werden, daß die erfindungsgemäßen Zusammensetzung, die das Glucanäther-Derivat als Wirkstoff aufweist, mit besonderem Erfolg auch als Anti-Aging-Mittel verwendbar ist. Insbesondere konnte beobachtet werden, daß bereits nach wenigen Tagen der Anwendung der erfindungsgemäßen Zusammensetzung eine Straffung der Haut, eine Reduktion der Faltentiefe und ein Glätten von rauher Haut eintrat, wobei die Anwender der erfindungsgemäßen Zusammensetzung übereinstimmend berichteten, daß sich die mit der erfindungsgemäßen Zusammensetzung behandelte Haut wesentlich elastischer und geschmeidiger anfühlte. Gleichzeitig reduzierte sich die Faltentiefe und Faltenhäufigkeit, insbesondere im Augenbereich, Wangenbereich und Oberlippenbereich. Ferner wurde festgestellt, daß sich bereits nach einem wiederholten Auftragen, so beispielsweise nach einem zweimaligen bis achtmaligen Auftragen, rauhe und spröde Haut schnell glättete, so daß dementsprechend die erfindungsgemäße Zusammensetzung auch zur Therapie von rauher Haut einsetzbar ist. Desweiteren berichteten Anwendern, daß rauhe und spröde Haut im Gegensatz zu früheren Erfahrungen selbst bei extremer Belastung der Haut nicht

auftrat, so daß dementsprechend die erfindungsgemäße Zusammensetzung auch zur Verhinderung des Auftretens von rauher und/oder spröder Haut, verwendbar ist.

Auch konnte festgestellt werden, daß die zuvor angesprochene Zusammensetzung sehr schnell nach dem Auftragen in die Haut einzieht, so daß ein unerwünschtes Nachfetten der Zusammensetzung bereits nach wenigen Sekunden bis einigen Minuten nach Applikation der Zusammensetzung verschwunden ist. Als sonstige Zusätze weist die zuvor angesprochene Zusammensetzung beispielsweise Verfestigungsmittel, so insbesondere hydrierte Polyisobutene, Konservierungsmittel, so beispielsweise Phenoxyethanol und/oder Imidazolidinyl-Harnstoff, Verdickungsmittel, so beispielsweise Stearinsäure und/oder Palmitinsäure, und Duftstoffe auf.

Das in der zuvor beschriebenen erfindungsgemäßen Zusammensetzung sowie den nachfolgend noch zu beschreibenden erfindungsgemäßen Zusammensetzungen enthaltene Wasser deckt alle wäßrigen Systeme ab, so insbesondere entionisiertes Wasser, destilliertes Wasser, wäßrige Salzlösungen sowie wäßrige Puffersysteme.

Eine bevorzugte Weiterbildung der zuvor beschriebenen topisch anwendbaren pharmazeutischen und/oder kosmetischen Zusammensetzung weist als Wirkstoff mindestens ein Glucanäther-Derivat auf, wobei dieser Wirkstoff chemisch so aufgebaut ist und so hergestellt wird, wie dies zuvor beschrieben ist. Hierbei beträgt vorzugsweise die Konzentration dieses Glucanäther-Derivates in der erfindungsgemäßen Zusammensetzung 0,04 Gew.%, wobei sich diese quantitative Angabe auf das Gewicht der anwendungsfertigen Zusammensetzung bezieht. Insbesondere wird bei der erfindungsgemäßen Zusammensetzung als Glucanäther-Derivat das Natriumsalz des Carboxymethylglucans verwendet, wobei dieses Natriumsalz des Carboxymethylglucans durch die Formel VI konkretisiert ist.

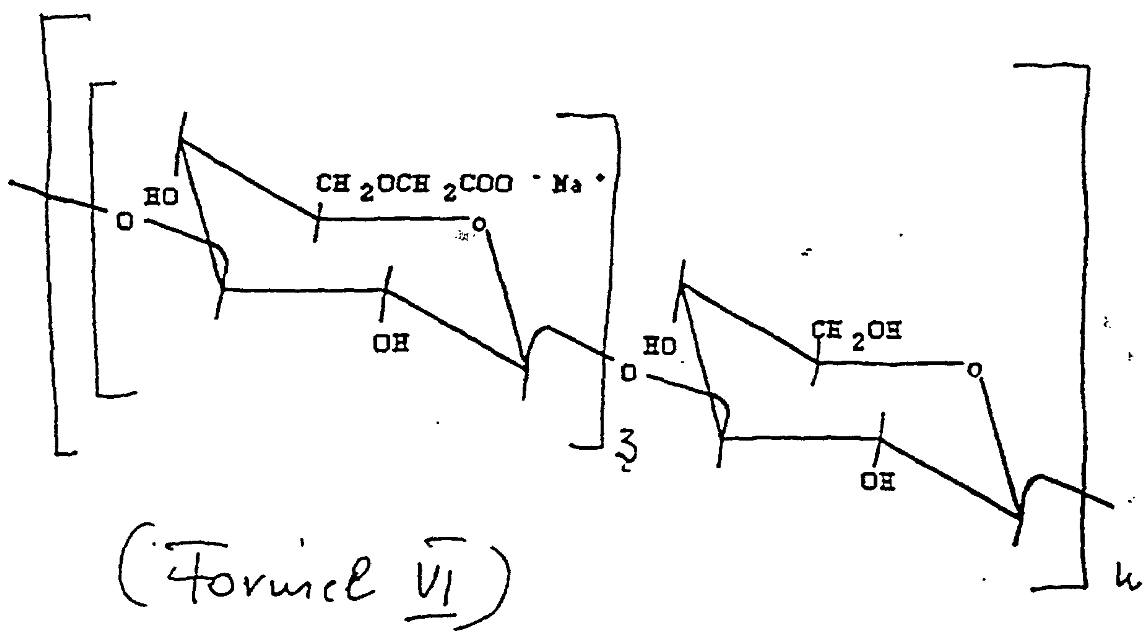

(Formel VI)

Wie bereits vorstehend ausgeführt ist, sind die erfindungsgemäßen pharmazeutischen und/oder kosmetischen Zusammensetzungen insbesondere zur Prophylaxe und/oder Therapie von durch oxidativen Streß in der Haut hervorgerufenen Erkrankungen, Reizungen und/oder Beschwerden verwendbar. Hierunter fallen auch solche Erkrankungen bzw. Reizungen und/oder Beschwerden, die im Laufe der Alterung der Haut auftreten. Konkret sind hier beispielsweise rauhe Haut, fehlende Elastizität, Faltenbildung, trockene Haut und die Hautschorfbildung zu benennen, wobei nachfolgend noch auf spezielle Verwendungen und Ausführungsformen der erfindungsgemäßen Zusammensetzung eingegangen wird.

Darüber hinaus konnte festgestellt werden, daß die erfindungsgemäße topisch anwendbare, pharmazeutisch und/oder kosmetische Zusammensetzung zur Wundheilung verwendbar ist. Nach einem einmaligen bis viermaligen Auftrag auf kleinere Wunden konnte beobachtet werden, daß die Bildung von neuem Gewebe verstärkt abläuft, ohne daß dabei Infektionen oder Entzündungen auftraten. Auch wurde die Narbenbildung beschleunigt, wobei die mit der erfindungsgemäßen Zusammensetzung behandelten Narben elastisch und reißfest waren. Bedingt durch die creme- bis gelartige Konsistenz der erfindungsgemäßen Zusammensetzung trat zudem noch eine angenehme Kühlung der Wunde auf, was insbesondere bei Verbrennungswunden als sehr angenehm empfunden wurde.

Insbesondere weist eine zur Wundheilung topisch anwendbare pharmazeutische und/oder kosmetische Zusammensetzung eine cremeartige bis gelartige Konsistenz auf und.enthält zwischen 0,002 Gew.% und 1 Gew.% des zuvor beschriebenen Glucanäther-Derivates, zwischen 1 Gew.% und 3 Gew.% Panthenol, zwischen 15 Gew.% und 28 Gew.% eines fett- bzw. ölartigen Zusatzes, so vorzugsweise Lecithin, Propylenglykol, Polyethylenglykolether höherer Fettalkohole sowie Triglyceride von höheren Fettsäuren, zwischen 0,05 Gew.% und 7 Gew.% sonstiger Zusätze, wie vorzugsweise Alkohole, insbesondere $C_1$-$C_4$-Alkohole, Vitamine, vorzugsweise Vitamin A und/oder Vitamin E und Parfum, sowie zwischen 83,948 Gew.% und 61 Gew.% Wasser.

Besonders gute Ergebnisse lassen sich mit einer Ausführungsform der erfindungsgemäßen Zusammensetzung im Bereich der Wundheilung dann erzielen, wenn diese Ausgestaltung der erfindungsgemäßen Zusammensetzung zwischen 0,1 Gew.% und 0,2 Gew.% des vorstehend in Formel VI gezeigten Natriumsalzes des Carboxymethyl-Glucans enthält. Hier konnte festgestellt werden, daß eine derartige Ausgestaltung der erfindungsgemäßen Zusammensetzung eine besonders rasche Wundheilung bewirkt, wobei unter den Begriff Wunde alle an sich bekannten kleinen Wunden mit und ohne Infektionsgefahr, wie beispielsweise Schürfungen, kleine Schnitt- und Kratzwunden, Schrunden, leichte Verbrennungen, Sonnenbrand, Wundsein sowie Entzündungen jeglicher Art fallen.

Neben den zuvor beschriebenen Anwendungen der erfindungsgemäßen Zusammensetzung konnte desweiteren überraschend festgestellt werden, daß sich die erfindungsgemäße Zusammensetzung auch zur Therapie und/oder Prophylaxe von Hautreizungen und/oder Hauterkrankungen, insbesondere zur Therapie und/oder Prophylaxe von Neurodermitis und/oder Psoriasis, eignet.

Besonders vorteilhaft für eine derartige Anwendung der erfindungsgemäßen Zusammensetzung im Bereich der zuvor genannten Hautreizungen und/oder Hauterkrankungen ist es, wenn die erfindungsgemäße Zusammensetzung neben dem Wirkstoff auf der Basis des zuvor beschriebenen Glucanäther-Derivates und Wasser noch mindestens ein Phospholipid sowie mindestens ein Öl aufweist.

Grundsätzlich kann die zuvor beschriebene Ausführungsform der erfindungsgemäßen Zusammensetzung, die somit als Mindestbestandteile Wasser, ein Phospholipid, ein Öl sowie ein Glucanäther-Derivat aufweist, in jeder, für die topische Anwendung geeigneten Darreichungsform vorliegen, wobei eine flüssige Formulierung als Emulsion bzw. Suspension und insbesondere eine Formulierung als Nanoemulsion bevorzugt wird. Im letzteren Fall stellt somit dann die erfindungsgemäße Zusammensetzung eine Nanoemulsion des mindestens einen Glucanäther-Derivates, des mindestens einen Phospholipids und des mindestens einen Öls in Wasser oder in einem Gemisch aus Wasser und Alkohol dar, wobei die Teilchengröße der emulgierten Teilchen vorzugsweise zwischen 30 nm und 5.000 nm, insbesondere zwischen 50 nm und 200 nm, variiert.

Bezüglich des in dieser Ausführungsform der erfindungsgemäßen Zusammensetzung enthaltenen Öls ist festzuhalten, daß es sich hierbei entweder um ein einziges Öl oder um ein Gemisch von Ölen, insbesondere um ein Gemisch von pflanzlichen flüssigen Ölen, handelt.

Vorzugsweise weist die zuvor beschriebene Ausführungsform der erfindungsgemäßen Zusammensetzung dann als Öl ein Gemisch von pflanzlichen Ölen auf, wobei dieses Gemisch von pflanzlichen flüssigen Ölen insbesondere Mandelöl, Avocadoöl, Calendulaöl und/oder Jojobaöl umfaßt.

Insbesondere dann, wenn die zuvor beschriebene, topisch anwendbare pharmazeutische und/oder kosmetische Zusammensetzung, die vorzugsweise als Nanoemulsion vorliegt, zwischen 0,05 Gew.% und 1 Gew.% des Glucanäther-Derivates, wie dieses vorstehend beschrieben und/oder wie dieses nach dem zuvor beschriebenen Verfahren hergestellt ist, zwischen 15 Gew.% und 25 Gew.% des Öls, insbesondere eines pflanzlichen Öls und vorzugsweise einer Mischung von Mandelöl, Avocadoöl, Calendulaöl und/oder Jojobaöl, zwischen 1 Gew.% und 5 Gew.% eines Phospholipids, insbesondere eines Phospholipids, das zwischen 50 Gew.% und 90 Gew.% Phosphatidylcholin enthält, zwischen 0 Gew.% und 8 Gew.% eines Alkohols, vorzugsweise eines $C_1$-$C_4$-Alkohols, zwischen 8 Gew.% und 15 Gew.% sonstiger Zusätze sowie zwischen 75,9 Gew.% und 46 Gew.% Wasser aufweist, besitzt eine derartige spezielle Zusammensetzung hervorragende therapeutische und/oder prophylaktische Eigenschaften. Unter den vorstehend verwendeten Begriff "sonstiger Zusätze" fallen insbesondere Konservierungsmittel, wie vorzugsweise Phenoxyethanol, Vitamine, wie vorzugsweise Vitamin E-Acetat, Glycerin und Parfum.

Wenn die zuvor beschriebene topisch anwendbare pharmazeutische und/oder kosmetische Zusammensetzung zur Therapie und/oder Prophylaxe von Hautreizungen und/oder Hauterkrankungen eingesetzt wird und wenn diese Zusammensetzung dann als Nanoemulsion vorliegt und insbesondere 0,2 Gew.% des Glucanäther-Derivates, vorzugsweise das in Formel VI wiedergegebene Natriumsalz des Carboxymethylglucans, enthält, läßt sich eine derartige spezielle Zusammensetzung hervorragend zur Behandlung von Neurodermitis sowie Psoriasis einsetzen, wie anhand einer ersten Studie, die nachfolgend noch beschrieben ist, festgestellt wurde.

Insgesamt ist somit festzuhalten, daß die zuvor beschriebenen erfindungsgemäßen Zusammensetzungen als topisch anwendbare pharmazeutische und/oder kosmetische Zusammensetzungen zur Behandlung von Hautreizungen bzw. Hauterkrankungen, die ihre Ursache in einem oxidativen Streß in der Haut haben, als Sonnenschutzmittel, als Sonnenmilch nach einer Bestrahlung, als Tagescreme, als Körpercreme und/oder als Reinigungszusammensetzungen mit hervorragenden Erfolgen anwendbar sind. Desweiteren konnte festgestellt werden, daß die erfindungsgemäßen Zusammensetzungen auch als Anti-Aging-Mittel einsetzbar und dabei insbesondere zur Straffung der Haut,

zur Reduktion der Faltentiefe und/oder zur Prophylaxe und/oder Therapie von rauher und/oder spröder Haut verwendbar sind. Ferner hat sich herausgestellt, daß die erfindungsgemäßen Zusammensetzungen zur Wundheilung und/oder zur Therapie und/oder Prophylaxe von Hautreizungen und/oder Hauterkrankungen, insbesondere zur Therapie und/oder Prophylaxe von Neurodermitis und/oder Psoriasis, mit großem Erfolg eingesetzt werden können.

Zuvor ist wiederholt beschrieben worden, daß die erfindungsgemäße Zusammensetzung als Wirkstoff Glucanäther-Derivate, insbesondere Carboxymethylglucan, enthält, wobei die entsprechenden Monomereneinheiten dieser Glucanäther-Derivate über eine 1,3-beta-glykosidische Bindung miteinander verknüpft sind. Diese Formulierung soll jedoch auch solche Derivate umfassen, bei denen die Monomereneinheiten linear über eine 1,3-beta-glykosidische Bindung und zusätzlich noch verzweigt über eine 1,6-beta-glykosidische Bindung verknüpft sind, wobei jedoch der Anteil der verzweigten 1,6-beta-glykosidischen Bindungen wesentlich geringer ist als der Anteil der linearen 1,3-beta-glykosidischen Bindungen und insbesondere zwischen 0 % und 20 %, vorzugsweise zwischen 4 % und 10 %, bezogen auf den Anteil der 1,3-glykosidischen Bindungen, ausmacht.

Die Erfindung wird nachfolgend anhand von Ausführungsbeispielen näher erläutert.

**Herstellung von Carboxymethyl-Glucan**

1 kg Glucan wurde in 20 1 Isopropanol unter Rühren in der Kälte suspendiert und mit 2 kg 30 Gew.%iger wäßriger Natriumhydroxidlösung während 16 Stunden bei Raumtemperatur (16°C - 22 °C) behandelt.

Nach Abschluß dieser alkalischen Behandlung wurde nach einer kurzen Verweilzeit von etwa 20 Minuten der über der Suspension anstehende Überstand von 8 1 des wäßrigen alkalischen Isopropanols abdekantiert. Hiernach erfolgte die Zugabe von 8 1 Isopropanol.

Unter Rühren wurde dann die Suspension auf 60 °C erwärmt.

Nach Erreichen der Endtemperatur wurden 1,5 kg des Natriumsalzes der Chloressigsäure, gelöst in 1,5 kg Wasser, zugesetzt.

Nach einer Gesamtreaktionszeit von 2,5 Stunden bei 60 °C wurde das Reaktionsprodukt sedimentiert und die darüber befindliche Flüssigkeit abdekantiert. Anschließend erfolgte ein Zusatz von 4 l einer wäßrigen Isopropanollösung (V:V, 50 % : 50 %).

Nachdem das Reaktionsprodukt gewaschen war, wurde das Natriumsalz des Carboxymethyl-Glucans in 20 l Wasser gelöst. Es erfolgte ein Zusatz zu der Lösung einer 10 N Salzsäure, derart, daß der pH-Wert der Lösung auf einen Wert von 7 eingestellt wurde.

Aus der neutralisierten, wäßrigen Lösung wurde das Natriumsalz des Carboxymethyl-Glucans durch Zusatz von 40 l Isopropanol ausgefällt.

Nach Abtrennung des ausgefällten Natriumsalzes des Carboxymethyl-Glucans wurde dieses im Vakuum-Trockenschrank bei 60 °C getrocknet.

Die Ausbeute an Natriumsalz des Carboxymethyl-Glucans betrug 1,3 kg.

Das Natriumsalz des Carboxymethyl-Glucans wurde analysiert. Hierbei zeigte sich, daß das Produkt einen Durchschnittssubstitutionsgrad von 0,75 ± 0,1 aufwies.

**In-Vivo-Test von 3 Formulierungen in bezug auf Ihre Wirksamkeit gegen den UVA-induzierten oxidativen Streß**

Es wurden drei unterschiedliche Creme formuliert, wobei die Creme folgende Zusammensetzungen aufwiesen:

| | Creme A |
|---|---|
| 1. Komponente | 0,5 % Phenoxyethanol<br>0,3 % Imidazolidinyl Urea<br>0,02 % Triethanolamine<br>2 % Alcohol<br>1 % Propylene Glycol<br>87,66 % Aqua |
| 2. Komponente | 4 % PEG-5 Glyceryl Stearate<br>1 % Cetyl Alcohol<br>1 % Stearic Acid<br>2 % Paraffinum Liquidum<br>0,5 % Hydrogeneted Polyisobutene |

(fortgesetzt)

| | Creme A |
|---|---|
| 3. Komponente | 0,02 % Parfum |
| | Creme B |
| 1. Komponente | 0,5 % Phenoxyethanol<br>0,3 % Imidazolidinyl Urea<br>0,02 % Triethanolamine<br>2 % Alcohol<br>1 % Propylene Glycol<br>0,2 % Sodium Carboxymethyl Betaglucan<br>87,46 % Aqua |
| 2. Komponente | 4 % PEG-5 Glyceryl Stearate<br>1 % Cetyl Alcohol<br>1 % Stearic Acid<br>2 % Paraffinum Liquidum<br>0,5 % Hydrogenated Polyisobutene |
| 3. Komponente | 0,02 % Parfum |
| | Creme C |
| 1. Komponente | 0,5 % Phenoxyethanol<br>0,3 % Imidazolidinyl Urea<br>0,02 % Triethanolamine<br>2 % Alcohol<br>1 % Propylene Glycol<br>0,04 % Sodium Carboxymethyl Betaglucan<br>87,62 % Aqua |
| 2. Komponente | 4 % PEG-5 Glyceryl Stearate<br>1 % Cetyl Alcohol<br>1 % Stearic Acid<br>2 % Paraffinum Liquidum<br>0,5 % Hydrogenated Polyisobutene |
| 3. Komponente | 0,02 % Parfum |

Die vorstehend wiedergegebenen %-Angaben beziehen sich alle auf Gewichtsprozent und die dort verwendeten Produktbezeichnungen entsprechen der INCI-Declaration, wie sie dem "International Cosmetic Ingredient Dictionary", 1995, veröffentlicht von "The Cosmetic, Toiletry, and Fragrance Association", Washington, zu entnehmen sind.

Zur Herstellung der Cremen A bis C wurde einheitlich wie folgt verfahren:

Die erste Komponente wurde jeweils in einen Reaktor eingefüllt und auf ca. 60 °C erwärmt. Unter Rühren wurden die auf etwa 70 °C erwärmte zweite Komponente zugegeben. Die vereinigten Komponenten wurden sodann in dem Reaktor homogenisiert. Anschließend erfolgte unter Rühren ein Abkühlen des Gemisches auf ca. 35 °C. Nach Erreichen dieser Temperatur wurde die dritte Komponente zugegeben und unter Rühren auf 25 °C abgekühlt.

Auf definierten Hautarealen (Durchmesser ca. 2,5 cm) der Unterarminnenseiten von 10 Probanden wurden für 5 Tage zweimal täglich ca. 2 $mg/cm^2$ die jeweilige Creme A bis Creme C appliziert. Zwei Felder blieben unbehandelt und dienten als Kontrolle.

Am fünften Tag erfolgte 30 Minuten nach der letzten Applikation der Creme A bis C eine Bestrahlung mit

UVA-Licht (10 $J/cm^2$; Strahlungsquelle: Multiport, Modell 601, Solar Light, USA). Ein Testfeld wurde jeweils nicht bestrahlt.

Nach Extraktion des Squalens und des Squalenhydroperoxids mit 1 ml Ethanol wurden hochdruckflüssigkeitschromatographisch die Konzentrationen des Squalens und des Squalenhydroperoxids in den Extrakten der unterschiedlich behandelten definierten Hautarealen bestimmt.

Die Squalen-Bestimmung erfolgte nach H.P. Nissen et al, Chromatographia 11/12; 686-690; 1990, wobei für die Bestimmung folgende HPLC-Bedingungen eingehalten wurden:

| | |
|---|---|
| Flußrate | 1 ml/min |
| Säule | LiChrospher RP 18 (5 µm) ; 4,6 x 125 mm |
| Eluent | Acetonitril/i-Propanol (50/50) |
| Detektion | 210 nm |

Die Bestimmung des Squalenhydroperoxids erfolgte nach J.R. Zhang et al, Free Radic. Biol. Med. 1: 1-10; 1995, A.E. Holley et al, Free Rad. Res. Comms. Vol. 15., No. 1., 51-63; 1991 und C. Colin et al, IFSCC-Konferenz 1994, Venedig, A 105, wobei eine Nachsäulenderivatisierung mit Isoluminol/Mikroperoxidase durchgeführt wurde, wobei die Detektion durch Chemilumineszenz erfolgte. Hierbei galten folgende Bedingungen:

| | |
|---|---|
| Flußrate | 1 ml/min |
| Säule | LiChrospher RP-Select B (5 µm); 4,6 x 125 mm |
| Eluent | Methanol |
| Derivatisierungsreagenz | Isoluminol (1 mM) und |
| | Mikroperoxidase (10 µg/ml) gelöst in 100 mM Borat-Puffer (pH 10)/Methanol (70/30) |
| Flußrate/ Derivatisierungsreagenz | 0,8 ml |
| Detektion | Chemilumineszenz |

Als Ergebnis der Untersuchung ist festzuhalten, daß in den Extrakten, die von den mit den Cremen B und C behandelten Hautarealen gewonnen wurden, nur geringe Mengen an Squalenhydroperoxids nachgewiesen werden konnten. Hingegen ließen sich in solchen Extrakten, die aus der mit der Creme A behandelten Hautareal sowie aus den unbehandelten Hautarealen nach Bestrahlung gewonnen wurden, größere Mengen an Squalenhydroperoxid analysieren.

Da Squalenhydroperoxid durch reaktive Sauerstoffspezies gebildet wird, kann aus diesen Ergebnissen geschlossen werden, daß die Creme B und C, die den Wirkstoff Natriumsalz des Carboxymethyl-Glucans enthalten, in der Lage sind, die Bildung von Squalenhydroperoxiden durch freie Radikale deutlich zu reduzieren.

In der folgenden Tabelle 1 sind die Ergebnisse der Untersuchung als prozentuale Verhinderung der Peroxidation der mit den Cremen B und C behandelten Arealen relativ zu dem mit der Creme A behandelten Areal ausgedrückt.

Tabelle 1

| Produkt | Verhinderung der Peroxidation (in %) bezogen auf die mit Creme A behandelten Hautarealen |
|---|---|
| Creme B | 94,9 |
| Creme C | 58,9 |

Die vorstehend in der Tabelle 1 wiedergegebene Verhinderung der Peroxidation wird wie folgt berechnet:

100 x (SQOOH (Creme A) - SQOOH (Creme B oder Creme C))/SQOOH

(Creme A)

SQOOH = Squalenhydroperoxidkonzentration/Squalenkonzentration

Weder bei der Creme B noch bei der Creme C konnte während der Anwendung bei den 10 Probanden, deren

Alter zwischen 18 Jahren und 40 Jahren betrug, irgendeine Hautunverträglichkeit oder eine Hautreizung beobachtet werden.

**In-Vivo-Test von zwei Formulierungen in bezug auf ihre Wirksamkeit als Anti-Aging-Mittel**

Zum Nachweis der Wirksamkeit von Glucanäther-Derivate enthaltenden Zusammensetzungen wurden zwei cremeartigen Formulierungen untersucht, wobei die nachfolgend wiedergegebene cremeartige Zusammensetzung mit der Bezeichnung 2LA als Wirkstoff das Natriumsalz des Carboxymethylbetaglucans, wie dieses vorstehend durch die Formel VI charakterisiert ist, enthält, während die ansonsten identisch aufgebaute cremeartige Zusammensetzung mit der Bezeichnung 2LB diesen zuvor genannten Wirkstoff nicht aufwies.

Die beiden untersuchten Zusammensetzungen 2LB und 2LA, die analog zum vorstehenden Beispiel hergestellt wurden, wiesen die nachfolgend aufgelisteten Inhaltsstoffe auf, wobei die Produktbezeichnungen der INCI-Declaration, wie sie dem zuvor genannten Dictionary zu entnehmen ist, entsprechen.

| INCI-Namen | 2LB Gew.% | 2LA Gew.% |
|---|---|---|
| Aqua | 87,5 | 87,46 |
| Alcohol | 2 | 2 |
| Propylene Glycol | 1 | 1 |
| Sodium Carboxymethyl Betaglucan | 0 | 0,04 |
| Phenoxyethanol | 0,5 | 0,5 |
| Imidazolidinyl Urea | 0,3 | 0,3 |
| PEG-5 Glyceryl Stearate | 4 | 4 |
| Cetylalcohol | 1 | 1 |
| Stearic Acid/Palmitic Acid | 1 | 1 |
| Paraffin | 2 | 2 |
| hydriertes Polyisobuten | 0,5 | 0,5 |
| Parfum | 0,2 | 0,2 |

Zehn gesunde weibliche Testpersonen in einem Alter zwischen 61 und 75 Jahren trugen zweimal täglich über einen Zeitraum von 28 Tagen auf ausgewählte Testgebiete auf den Unterarminnenseiten und im Gesicht (Augenfalten) die zuvor beschriebenen Zusammensetzungen 2LA und 2LB auf.

Zu Beginn der Studie wurde vor der ersten Applikation der Zusammensetzungen die Hautelastizität, die Faltentiefe sowie die Hautrauhigkeit gemessen, wobei diese zuvor genannten drei Parameter nach 14 Tagen und 28 Tagen nach Applikation wiederholt wurden.

Die Testpersonen wurden angewiesen, drei Tage vor Beginn der Prüfung und während der gesamten Testzeit (28 Tage) keine anderen Externa auf die Testgebiete aufzutragen. Zur Reinigung durfte in den Testgebieten nur Wasser verwendet werden.

Die letzte Messung nach 28 Tagen erfolgte 8 Stunden nach der letzten Applikation des jeweiligen Produktes.

Die Testareale der Unterarminnenseiten wurden zweimal pro Woche mit einem Sonnensimulator mit 0,75 MED (minimale Erythemdosis) bestrahlt. Die zuvor angesprochenen Parameter wurden wie folgt gemessen:

a) Messung der Hautelastizität (Hautstraffung)

Die Elastizität der Haut wurde mit dem Cutometer SEM 474 (Hersteller: Courage & Khazaka Electonic GmbH, Köln) gemessen. Hierbei wurde die Hautoberfläche durch ein angelegtes Vakuum in die Öffnung (2 mm, innerer Durchmesser) einer speziellen Sonde gezogen. Die Eindringtiefe der Haut in die Sonde wurde ohne mechanische Einwirkung reibungslos optisch erfaßt. Die Messungen wurden mit konstanten Unterdruck (500 mbar) bei einer Meßzeit von 1 Sekunde durchgeführt. Anschließend wurde der Unterdruck für 1 Sekunde abgeschaltet und der Meßzyklus dann noch fünfzigmal wiederholt.

Zur Auswertung wurden die 50 maximalen und 50 minimalen Amplituden verwendet. Die Datensätze (für jeden Probanden und jeden Meßtag) wurden dann an eine logarithmische Funktion des Typs $y = 1/a (\ln x + b)$ angepaßt, wobei x = maximale Amplitude bzw. minimale Amplitude und y = Anzahl der Meßzyklen entspricht. Die Anpassung geschah durch die Minimierung der Summe der Restwerte für die angepaßte Kurve (Programm:Statgraphics Plus for Windows-Version 3.0 - Manugistics, USA).

Bei allen Anpassungen war der Betrag des Korrelationskoeffizienten größer als 0,9. Die Werte von a und b der individuellen logarithmischen Funktionen wurden dann für jedes Testareal und jeden Meßzeitpunkt gemittelt.

Aus den Mittelwerten wurden die logarithmischen Funktionen gezeichnet und miteinander verglichen. Die Flächen unter den Funktionen wurden durch Bildung des Integrals zwischen 0 und 100 nach

$$y = \int (\ln x + b)/a \, dx = (bx + x\ln x) /a$$

sowohl für die Maxima als auch für die Minima berechnet (Progamm:Mathcad 6.0 Mathsoft, USA).

b) Messung der Faltentiefe

Die Bestimmung der Faltentiefe erfolgt mit dem Hautoberflächenmeßgerät OFR 01 (Romano GmbH, Köln). Die Bestimmung der Faltentiefe erfolgte mit Hilfe der Profilometrie. Zur Beschreibung der Faltentiefe wird der Parameter Rt (DIN 4768/1) gewählt. Rt ist die maximale Rauhtiefe, d.h. der Abstand zwischen der Linie der Erhebung und der Linie der Vertiefung innerhalb der Meßstrecke. Da eine direkte Messung an den Hautoberflächen nicht möglich ist, wurden entsprechende Hautabdrücke mit Silasoft N (Hersteller: Detax-Dentol, Karlsruhe) angefertigt und unter den nachfolgenden Bedingungen vermessen:

| | |
|---|---|
| Meßweg | 15.000 µm |
| auswertbarer Weg | 12.500 µm |
| Schrittweite | 2,5 µm |
| Gesamtschritte | 5.000 |
| Zahl der Messungen pro Abdruck | 11 |

c) Messung der Hautrauhigkeit

Die Bestimmung der Hautrauhigkeit erfolgte mit dem Skin Visiometer VS 400 (Courage & Khazaka Electronic GmbH, Köln).

Das Meßprinzip beruht auf der Durchleuchtung eines sehr dünnen, speziell angefärbten Silikonhautabdrukkes. Das Licht wird dabei von dem Silikonmaterial, je nach dessen Stärke, entsprechend absorbiert. Der Silikonhautabdruck bildet die Höhen und Tiefen der Haut als Negative ab, d.h. Hautfältchen sind im Abdruck höher, dementsprechend ist das Silikonmaterial an dieser Stelle dicker. Die Meßmethode basiert auf dem Bouguer-Lambertschen Absorptionsgesetz.

Durch Sichtbarmachung der Lichtabsorption, z.B. durch eine schwarz/weiß CCD-Kamera auf einem PC-Monitor, ist es möglich, die Höhe und Tiefen jedes Punktes des Abdruckes durch eine entsprechende Einstufung in eine Grauwert-Skala darzustellen. Die Entfernung eines jeden Punktes zur Grundlinie des Hautabdruckes kann durch die speziell entwickelte Bild-Digitalisierungstechnik und die Software des Skin Visiometers VS 400 in mm berechnet werden.

Die so gewonnenen Meßdaten wurden zentral nach eingehender Plausibilitätsprüfung und Qualitätssicherung in einen Rechner übernommen. Die Auswertung wurde mit Hilfe der Software "Statgraphics" für Windows, Version 3.0 - Manugistics, USA durchgeführt. Neben der deskriptiven Statistik wurde der "Wilcoxon matched pairs signed rank test" eingesetzt, wobei $p \leq 0,05$ als Hinweis auf einen statistischen Unterschied gilt.

Als Ergebnis der zuvor angesprochenen Untersuchung ist festzuhalten, daß die Anwendung des Produktes 2LA nach 28 Tagen zu einer statistisch signifikanten Straffung der Haut gegenüber der Ausgangssituation und gegenüber der unbehandelten Situation führt. Weiterhin bewirkt die Anwendung der Zusammensetzung 2LA eine statistisch signifikante Reduktion der Faltentiefe gegenüber der Ausgangssituation, während sich die Hautrauhigkeit ebenfalls statistisch gesichert vermindert. Desweiteren zeigen die Testergebnisse deutlich, daß die Zusammensetzung 2LA der Zusammensetzung 2LB deutlich überlegen ist.

Diese zuvor wiedergegebenen Aussagen werden durch die Abbildungen 1 bis 3 gemäß EP 0 819 703 A2 quantifiziert.

**In-Vivo-Test von einer Formulierung in bezug auf seine Wirksamkeit zur Behandlung von Psoriasis und Neurodermitis**

Es wurde eine Mikroemulsion hergestellt, die die folgenden Inhaltsstoffe aufwies:

| INCI-Namen | Gew.% |
|---|---|
| Lecithin | 2,5 |

(fortgesetzt)

| INCI-Namen | Gew.% |
|---|---|
| Alcohol | 6 |
| Sweet Almond Oil | 10 |
| Avocado Oil | 5 |
| Calendula Oil | 5 |
| Tocopheryl Acetat | 0,1 |
| Glycerine | 10 |
| Aqua | 60,6 |
| Propylene Glycol | 0,5 |
| Phenoxyethanol | 0,1 |
| Sodium Carboxymethyl Betaglucan | 0,2 |
| Parfum | 0,01 |

Die zuvor wiedergegebenen Bezeichnungen der Inhaltsstoffe erfolgte nach der INCI-Declaration, wie diese dem zuvor zitierten Dictionary zu entnehmen ist.

Mit dieser, nachfolgend auch als Zusammensetzung D bezeichneten Formulierung wurde zunächst eine 31 Jahre alte Frau behandelt, wobei diese Patientin an Psoriasis guttata erkrankt ist. Hierbei waren der rechte Handrücken, der linke kleine Finger, beide Beine (Innen- und Außenseite mehrere ca. 1 cm große Stellen), das linke Bein (5 cm große Stelle oberhalb des Fußinnenknöchels, entstanden nach starker, zweigradiger Verbrennung) sowie der gesamte Oberkörper mit kleineren Herden befallen, wobei zusätzlich noch beide Ellenbogen stark verkrustet waren.

Zur Behandlung wurden die befallenen Herde mehrmals nacheinander eingecremt, bis ein Sättigungsgefühl der Haut erreicht wurde.

Bereits nach einer viertägigen Behandlung waren die auf dem rechten Handrücken befallenen Stellen mit Ausnahme einer ganz kleinen Stelle ausgeheilt. An beiden Beinen war ein deutlicher Rückgang der Hautrötung sowie des Befalls feststellbar. Der 5 cm große Herd zeigte ebenfalls eine deutliche Heilung und einen deutlichen Rückgang der Rötung.

Die Patientin berichtete über ein subjektiv empfundenes gutes Hautgefühl und über ihre geschmeidige Haut nach Anwendung der Zusammensetzung D.

Eine 43 Jahre alte Frau, die seit etwa 30 Jahren an Psoriasis erkrankt ist und die in den letzten 10 Jahren nur noch einzelne, sporadisch auftretende Herde der Erkrankung am Ellenbogen und der Kopfhaut aufwies, berichtete, daß sie durch Anwendung der Zusammensetzung D auf den sporadisch auftretenden Psoriasisbefall innerhalb von wenigen Tagen eine nahezu sofortige Heilung erzielte. Dieses positive Ergebnis veranlaßte die Patientin, die früher eingesetzte Cortisonsalbe nicht mehr anzuwenden, da die Zusammensetzung D bei einem plötzlich auftretenden Psoriasisbefall eine bessere Abhilfe brachte als die ursprünglich verwendete Cortisonsalbe.

Ein 33 Jahre alter Mann, der über die Stirn verteilte, ca. 1 cm große entzündliche und schuppige Flecken, die sich bis in den Haaransatz und hinter die Ohren erstrecken, aufwies, verwendete etwa 2 Monaten die Zusammensetzung D. Hierbei erfolgte die Anwendung der Zusammensetzung D täglich nach dem Duschen. Bereits nach einwöchiger Anwendung der Zusammensetzung D konnte festgestellt werden, daß die entzündlichen, schuppigen Flecken nahezu spurlos verschwunden waren, wobei nach Abbruch der Behandlung mit der Zusammensetzung D nahezu unmittelbar erneut diese entzündlichen Herde auftraten. Desweiteren berichtete der Patient, daß nach Auftragen der Zusammensetzung D eine deutliche Linderung des Juckreizes zu bemerken war, wobei desweiteren die Anwendung der Zusammensetzung D dem Patienten ein deutliches positives Hautgefühl vermittelte.

Ein 17-jähriger Mann mit einer deutlich am Körper feststellbaren Neurodermitis wurde während 2,5 Wochen täglich zweimal mit der Zusammensetzung D behandelt. Nach Ablauf dieser Behandlungszeit war die Neurodermitis restlos verschwunden. Sobald jedoch die Behandlung mit der Zusammensetzung D beendet wurde, trat der Neurodermitis-Befall sofort wieder auf. Dies führte dazu, daß der Patient die entsprechende Behandlung fortführte, was wiederum innerhalb von kürzester Zeit eine deutliche Heilung bewirkte.

Eine für die Wundheilung verwendbare cremeartige Zusammensetzung weist insbesondere die nachfolgend wiedergegebenen Inhaltsstoffe auf:

| INCI-Namen | Gew.% |
|---|---|
| Aqua | 70 |
| Sodium Carboxymethyl Betaglucan | 0,1 |

(fortgesetzt)

| INCI-Namen | Gew.% |
|---|---|
| Panthenol | 2 |
| Propylene Glycol | 0,25 |
| Alcohol | 0,5 |
| Glycerine | 1 |
| Pentylenglycol | 1 |
| Methylchloroisothiazolinone | 0,1 |
| Allantoin | 0,1 |
| Disodium EDTA | 0,1 |
| Diisopropyl Adipate | 3 |
| Rentinyl Acetate | 0,5 |
| Lecithin | 0,2 |
| Caprylic/Capric Triglyceride | 9 |
| Steareth-2 | 3 |
| Steareth-21 | 2 |
| Tocopheryl Acetat | 2 |
| Farnesol | 1 |
| Stearic Acid/Palmitic Acid | 1,5 |
| Polyacrylamide | 2,5 |
| Parfum | 0,15 |

Die vorstehend verwendeten Bezeichnungen entsprechen der Declaration, wie sie dem "International Cosmetic Ingredient Dictionary", 1995, veröffentlicht von "The Cosmetic, Toiletry, and Fragrance Association", Washington, zu entnehmen sind.

**Patentansprüche**

1. Pharmazeutische und/oder kosmetische Zusammensetzung, wobei die Zusammensetzung neben Wasser mindestens einen Wirkstoff enthält, der mindestens ein polymeres Glucanäther-Derivat ist, das neben Monomereneinheiten der Formel I auch solche Monomereneinheiten der allgemeinen Formel II aufweist,

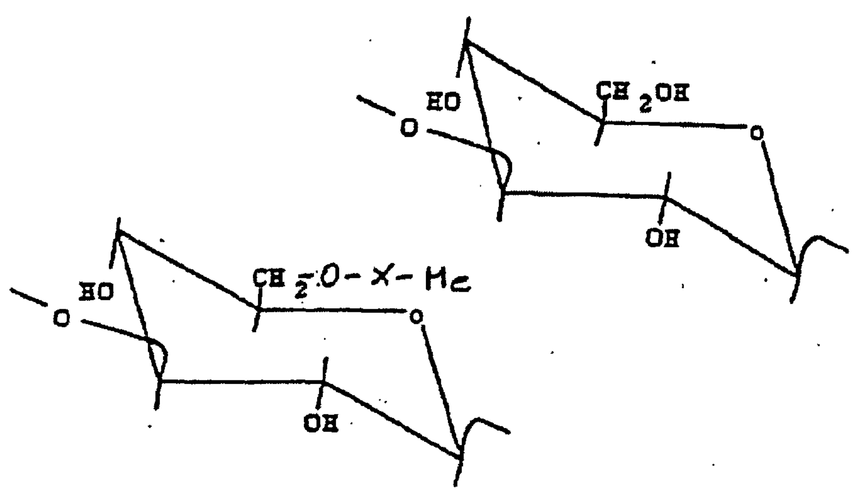

(Formel I)

(Formel II)

wobei die Monomereneinheiten der Formel I mit den Monomereneinheiten der Formel II über eine 1,3-beta-glykosidische Bindung verknüpft sind und wobei in Formel II
X eine $-CH_2-COO$-Gruppe, eine $-CH_2-CH_2-COO$-Gruppe, eine

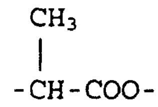

Gruppe und/oder eine $-CH_2-CH_2-SO_3$-Gruppe und
Me Wasserstoff, ein Alkali- und/oder ein Erdalkalimetall bedeuten, **dadurch gekennzeichnet, daß** die topisch applizierbare Zusammensetzung als Inhaltsstoffe

zwischen 0,002 Gew.% und 8 Gew.% des Glucanäther-Derivates,
zwischen 0 Gew.% und 3 Gew.% eines Alkohols, ausgenommen Alkylenglykol,
zwischen 0,5 Gew.% und 15 Gew.% eines Alkylenglykols,
zwischen 1 Gew.% und 3 Gew.% eines Paraffins,
zwischen 5,5 Gew.% und 9,5 Gew.% sonstiger Zusätze sowie
zwischen 92,988 Gew.% und 75 Gew.% Wasser enthält.

2. Zusammensetzung nach Anspruch 1, **dadurch gekennzeichnet, daß** die Zusammensetzung als Alkohol zwischen 0 Gew.% und 3 Gew.% Ethanol enthält.

3. Zusammensetzung nach Anspruch 1 oder 2, **dadurch gekennzeichnet, daß** die Zusammensetzung das Glucanäther-Derivat in einer Konzentration zwischen 0,02 Gew.% und 4 Gew.% enthält.

4. Zusammensetzung nach einem der vorangehenden Ansprüche, **dadurch gekennzeichnet, daß** die Zusammensetzung als Wirkstoff ein solches polymeres Glucanäther-Derivat enthält, das mindestens eine Monomereneinheit der nachfolgenden Formel IV

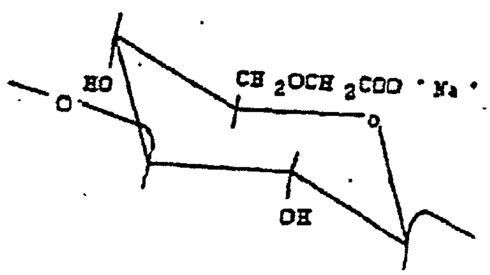

(Formel IV)

aufweist.

5. Zusammensetzung nach einem der vorangehenden Ansprüche, **dadurch gekennzeichnet, daß** das Glucanäther-Derivat einen Durchschnittssubstitutionsgrad von $0,75 \pm 0,1$ aufweist.

6. Zusammensetzung nach Anspruch 4 oder 5, **dadurch gekennzeichnet, daß** die Zusammensetzung als Glucanäther-Derivat 0,04 Gew.% des Carboxymethylglucan-Natriumsalzes gemäß Formel IV aufweist.

7. Zusammensetzung nach einem der vorangehenden Ansprüche zur Verwendung in der Therapie und/oder Prophylaxe von Neurodermitis und/oder Psoriasis, als Anti-Aging-Mittel zur Straffung der Haut, zur Reduktion der Faltentiefe und/oder zur Prophylaxe und/oder Therapie von rauher Haut.

## Claims

1. A pharmaceutical and/or cosmetical composition, whereby the composition contains beside water at least one active substance which is at least one polymer glucan ether derivative, containing in addition to the monomer units of the formula I also such monomer units of the general formula II

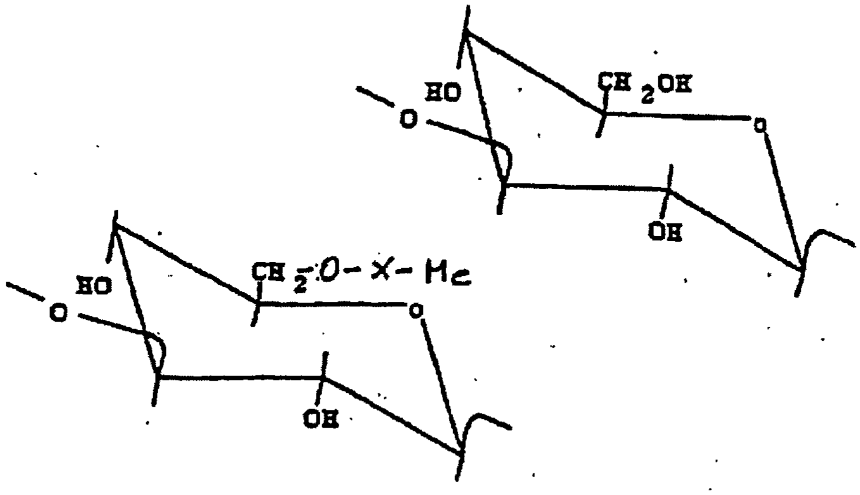

(Formel I)

(Formel II)

whereby the monomer units of the formula I are connected with the monomer units of the formula II by a 1,3-beta-glycosidic bond and whereby in formula II
X means a $-CH_2-COO$-group, a $-CH_2-CH_2-COO$-group, a

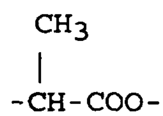

group and/or a $-CH_2-CH_2-SO_3$-group and
Me means hydrogen, an alkali- and/or an alkaline-earth metal, **characterised in that** the topical applicable composition contains as ingredients

between 0,002 % by weight and 8 % by weight of the glucan ether derivative
between 0 % by weight and 3 % by weight of an alcohol, except alkylenglycol,
between 0,5 % by weight and 15 % by weight of an alkylenglycol
between 1 % by weight and 3 % by weight of a paraffin
between 5,5 % by weight and 9,5 % by weight of other additives
and
between 92,988 % by weight and 75 % by weight of water.

**2.** The composition according to claim 1, **characterised in that** the composition contains ethanol as alcohol between 0 % by weight and 3 % by weight.

**3.** The composition according to claim 1 or 2, **characterised in that** the composition contains said glucan ether derivative in a concentration between 0,02 % by weight and 4 % by weight.

**4.** The composition according to one of the proceeding claims, **characterized in that** the composition contains such a polymer glucan ether derivative as active substance, which at least comprises one monomer unit of the following formula IV.

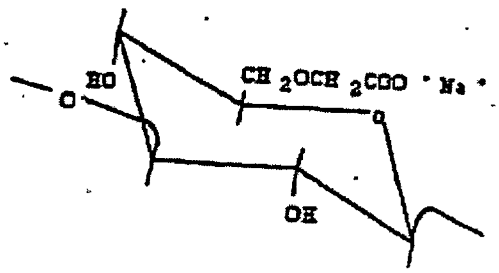

(Formel IV)

5. The composition according to one of the proceeding claims, **characterised in that** the glucan ether derivative has a average degree of substitution of 0,75 ± 0,1.

6. The composition according to claim 4 or 5, **characterised in that** the composition contains as glucan ether derivative 0,04 % by weight of carboxymethyl-glucan-sodium salt corresponding to formula IV.

7. The composition according to one of the proceeding claims being used for the therapy and/or the prophylaxis of neurodermatitis and/or of psoriasis, as anti-aging remedy for tightening the skin, for the reduction of the fold depth and/or for the prophylaxis and/or the therapy of chapped skin.

**Revendications**

1. Composition pharmaceutique et/ou cosmétique, la composition contenant, outre de l'eau, au moins un agent actif, qui est au moins un dérivé polymère d'éther de glucane qui, outre des unités monomères de la formule I, contient aussi des unités monomères de la formule générale II,

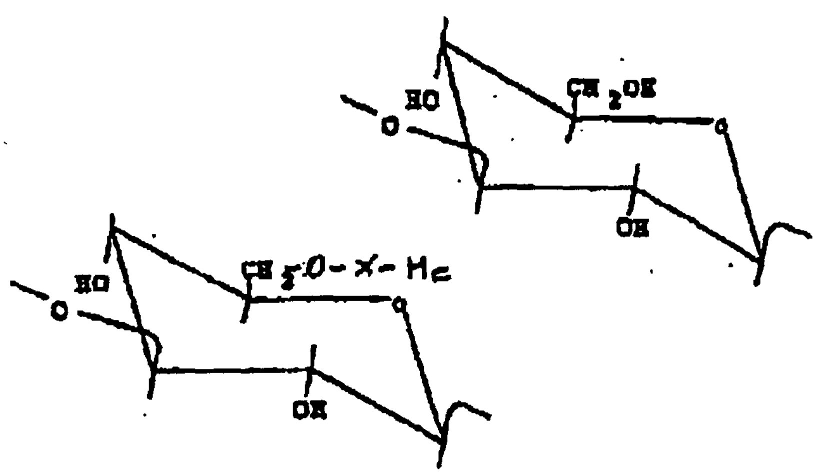

(formule I)

formule ( II)

où les unités monomères de la formule I sont liées aux unités monomères de la formule II par une liaison 1,3-β-glycosidique et où, dans la formule II,
X représente un groupe $-CH_2-COO$, un groupe $-CH_2-CH_2-COO$, un groupe $-CH(CH_3)-COO$ et/ou un groupe $-CH_2-CH_2-SO_3$ et
Me représente de l'hydrogène, un métal alcalin et/ou alcalino-terreux, **caractérisée en ce que** la composition applicable localement contient comme ingrédients:

de 0,002% en poids à 8% en poids du dérivé d'éther de glucane,
de 0% en poids à 3% en poids d'un alcool, à l'exception d'un alkylène glycol,

de 0,5% en poids à 15% en poids d'un alkylène glycol,
de 1% en poids à 3% en poids d'une paraffine,
de 5,5% en poids à 9,5% en poids d'autres additifs ainsi que
de 92,988% en poids à 75% en poids d'eau.

**2.** Composition selon la revendication 1, **caractérisée en ce que** la composition contient, en tant qu'alcool, de 0% en poids à 3% en poids d'éthanol.

**3.** Composition selon la revendication 1 ou 2, **caractérisée en ce que** la composition contient le dérivé d'éther de glucane en une concentration de 0,02% en poids à 4% en poids.

**4.** Composition selon l'une quelconque des revendications qui précèdent, **caractérisée en ce que** la composition contient comme agent actif un dérivé polymère d'éther de glucane présentant au moins une unité monomère de la formule IV ci-dessous:

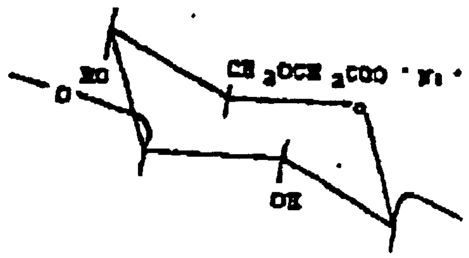

(formule IV)

**5.** Composition selon l'une quelconque des revendications qui précèdent, **caractérisée en ce que** le dérivé d'éther de glucane présente un degré moyen de substitution de 0,75 ± 0,1.

**6.** Composition selon la revendication 4 ou 5, **caractérisée en ce que** la composition présente comme dérivé d'éther de glucane 0,04% en poids du sel de sodium du carboxyméthylglucane selon la formule IV.

**7.** Composition selon l'une quelconque des revendications qui précèdent pour utilisation dans la thérapie et/ou la prophylaxie de la neurodermite et/ou du psoriasis, comme agent anti-âge pour le lissage de la peau, pour la réduction de la profondeur des rides et/ou pour la prophylaxie et/ou la thérapie de la peau rugueuse.